# EUROPÄISCHE PATENTSCHRIFT

(11) **EP 1 035 059 B1**
(45) Veröffentlichungstag und Bekanntmachung des Hinweises auf die Patenterteilung: **06.05.2004**
(21) Anmeldenummer: 00103031.1
(22) Anmeldetag: 15.02.2000
(51) Int. Cl.: B65H 63/06, G01B 11/00, G01B 11/10

(54) **Vorrichtung zur optischen Garnüberwachung**
Device for optical yarn monitoring
Dispositif pour le contrôle optique d'un fil

(30) Priorität: 05.03.1999 DE 19909703
(43) Veröffentlichungstag der Anmeldung: 13.09.2000
(73) Patentinhaber: Saurer GmbH & Co. KG, 41069 Mönchengladbach (DE)
(72) Erfinder: Henze, Herbert, 41063 Mönchengladbach (DE); Birlem, Olav, 41366 Schwalmtal (DE)

(56) Entgegenhaltungen:
- WO-A-00/07921
- DE-A- 2 621 900
- DE-A- 3 112 812
- DE-B- 1 798 297
- DE-U- 7 435 828
- US-A- 5 421 529

## Beschreibung

Die Erfindung betrifft eine Vorrichtung zur optischen Garnüberwachung.

Bei Spinn- und Spulprozessen fallen erhebliche Mengen von Staubteilchen, Faserfragmenten sowie Avivage- und Schmutzpartikeln an, die in der Luft schweben und von denen sich eine Teilmenge auf den Oberflächen der Bauteile der Spinnund Spulmaschinen ablagert. Das Freisetzen derartiger Partikel wird u.a. von der Reibung begünstigt, der der laufende Faden an einigen Stellen im Fadenlauf, zum Beispiel an Fadenführern, ausgesetzt ist. Die Bauteile von Garnüberwachungseinrichtungen befinden sich besonders nah am laufenden Faden und sind nach relativ kurzer Zeit mit derartigen Ablagerungen bedeckt. Dies hat nachteilig zur Folge, daß diese Oberflächen bald einen Verschmutzungsgrad erreichen, bei dem nur noch geschwächte oder überhaupt keine Signale mehr von der Garnüberwachungseinrichtung detektiert werden. Dieser Zustand führt zu verfälschten Meßergebnissen oder zum Ausfall der Messungen. Um das zu vermeiden, wird üblicherweise in bestimmten Zeitabständen eine vorbeugende Reinigung der Oberflächen der Bauteile, wie zum Beispiel der Lichtquellen, Sensoren oder deren lichtdurchlässigen Abdeckungen, vorgenommen.

Die DE 1 798 297 B zeigt eine Einrichtung zum Messen des Durchmessers und der Masse eines Fadens. Die Meßeinrichtung weist einen Meßkopf eines foto-elektrischen Fadenprüfers auf. Dabei schützen Platten aus durchsichtigem Material die Linsen, die emittiertes Licht in ein paralleles Strahlenbündel umwandeln oder Lichtstrahlen auf einen Brennpunkt bündeln, vor Verschmutzung. Diese Platten sind allerdings nun ihrerseits starker Verschmutzung ausgesetzt. Um die durch Verschmutzung beziehungsweise verminderte Lichtdurchlässigkeit der Plattenoberfläche hervorgerufene Verfälschung der Meßwerte zu begrenzen, werden die Platten im Rahmen von vorbeugenden Wartungsmaßnahmen gereinigt.

Dadurch bedingte kurze Reinigungsintervalle bedeuten relativ häufige Betriebsunterbrechung und führen damit zu einem schlechten Nutzungsgrad der Spinn- oder Spulmaschine. Durch die zahlreichen Reinigungsvorgänge wird die intermittierend gereinigte Oberfläche zunehmend verkratzt und die Lichtdurchlässigkeit bleibend herabgesetzt. Führt das Verkratzen der Oberfläche zu einem Zustand, bei dem eine fehlerfreie Messung nicht mehr gesichert ist, muß das Bauteil ausgetauscht werden. Somit vermindert die Verschmutzung die Dauer der einwandfreien Funktion beziehungsweise die Lebensdauer eines solchen Bauteiles erheblich. Dies erzeugt Austauschkosten und führt zu weiterer Senkung des Nutzungsgrades der Maschine durch die mit dem Austausch verbundenen Wartungsarbeiten und Stillstandszeiten.

Die DE 26 21 900 A beschreibt einen optischen Fadenwächter einer Spinnmaschine, der einen Lichtsender und einen Lichtempfänger aufweist. Um diese staubempfindlichen Bauteile vor der Verstaubung durch Faser- und Schmutzteile zu schützen, ist sowohl der Lichtsender als auch der Lichtempfänger durch eine lichtdurchlässige Abdeckung vom umlaufenden Faden beziehungsweise der Umgebung abgekapselt. Der Faden vollführt beim laufenden Spinnprozeß eine Kreisbewegung und wird dabei so über die Abdeckung gezogen, daß er beim Darübergleiten mit der Abdeckung Kontakt hat und diese dadurch von Schmutz befreit. Die Reibung zwischen Faden und lichtdurchlässiger Abdeckung führt besonders durch die Einwirkung der härteren Schmutzbestandteile nach relativ kurzer Nutzungsdauer zu einem Verkratzen der kontinuierlich von Schmutz befreiten Oberfläche ("Erblinden") der Abdeckung, so daß der Strahlendurchgang auch bei dieser Garnüberwachungseinrichtung bleibend behindert wird. Dies beeinträchtigt die Garnüberwachungsfunktion und führt zu den oben beschriebenen Nachteilen, insbesondere zu höheren Kosten und herabgesetztem Nutzungsgrad. Zudem ist ein derartiger Fadenwächter nicht beliebig einsetzbar, da die Voraussetzung für die oben beschriebene Reinigung durch das bewegte Garn eine Changierbewegung oder eine kreisförmige Umlaufbewegung des Fadens ist und diese Voraussetzung nicht an jeder für die Garnüberwachung gewünschten beziehungsweise erforderlichen Stelle des Fadenlaufes vorliegt.

Das DE 74 35 828 U offenbart ein berührungsfrei arbeitendes, als verschmutzungsunempfindlich bezeichnetes optisches Durchmessermeßgerät. Um das optische Durchmessermeßgerät über längere Zeit gegen die unter normalen Arbeitsbedingungen auftretenden Verschmutzungen unempfindlich zu machen, durchlaufen an der Meßoptik kontinuierlich oder intermittierend vorbeiführbare, als Schutzgläser beziehungsweise -folien ausgebildete Schutzeinrichtungen ein Reinigungsbad. Wird nach Durchlaufen des Reinigungsbades zum Entfernen von Flüssigkeitstropfen oder -schlieren ein fester Wischer beziehungsweise Abstreifer eingesetzt, tritt wiederum ein, wenn auch begrenzter, Oberflächenverschleiß auf. Alternativ sind die Schutzgläser beziehungsweise -folien einer Berieselungsvorrichtung zugeordnet, die diese Schutzeinrichtungen mit einer Reinigungsflüssigkeit überzieht. Auf solche Weise werden auftretende Verschmutzungen durch die herabrinnende Flüssigkeit direkt weggespült oder können sich gar nicht erst festsetzen.

Die WO 0 007 921 A, die nicht rechtzeitig veröffentlicht ist, offenbart eine Vorrichtung zur optischen garnüberwachung mit einer Einrichtung, die der Minderung der Lichtdurchlässigkeit durch Umgebungseinflüsse auf Bauelemente wie Lichtquelle, Sensor oder Linse, entgegenwirkt, wobei die Einrichtung gegen unerwünschte Folgen von Umgebungseinflüssen eine Lichtdurchlässige Beschichtung ist, die das Anlagern von die Lichtdurchlässigkeit mindernden Ablagerungen herabsetzt und den Verschleiß an der Oberfläche der Bauelemente infolge mechanischer Einwirkung mindert.

Dieses optische Durchmessermeßgerät hat einen ganz erheblichen Platzbedarf und erfordert hohen Aufwand für die mechanischen Einrichtungen und die Durchführung der Reinigungsfunktionen. Es entstehen beträchtliche Kosten durch die Bauteile und die Bereitstellung der Antriebsenergie für die Bewegungen der Schutzeinrichtungen beziehungsweise für die Reinigungseinrichtungen. Der hohe Aufwand und insbesondere der erhebliche Platzbedarf machen eine derartige Vorrichtung für die Garnüberwachung in einer Vielstellen-Textilmaschine ungeeignet.

Der Erfindung liegt die Aufgabe zugrunde, Vorrichtungen zur optischen Garnüberwachung, bei denen mindestens eine im Strahlengang optisch wirksame Oberfläche in der Lichtdurchlässigkeit durch Umgebungseinflüsse gemindert wird, zu verbessern.

Diese Aufgabe wird erfindungsgemäß durch eine Vorrichtung mit den Merkmalen des Anspruches 1 gelöst.

Vorteilhafte Ausgestaltungen der Erfindung sind Gegenstand der abhängigen Ansprüche.

Eine erfindungsgemäße lichtdurchlässige Beschichtung benötigt keinen zusätzlichen Bauraum und ist daher auch bei kompakten, raumsparend konstruierten Garnüberwachungsvorrichtungen einsetzbar. Eine derartige Beschichtung läßt sich kostengünstig ohne die Notwendigkeit zusätzlicher Bauteile beziehungsweise zusätzlicher Bauelemente herstellen, verbessert die Meßergebnisse und erhöht die Zuverlässigkeit der Garnüberwachungsvorrichtung. Es sind längere Wartungsbeziehungsweise Reinigungsintervalle möglich, und die Zahl der zustandsabhängigen und der störungsabhängigen Wartungsbeziehungsweise Reinigungsvorgänge verringert sich. Weniger Betriebsunterbrechungen führen zu einer Erhöhung des Nutzungsgrades der Maschine. Durch die Verlängerung der Lebensbeziehungsweise der Nutzungsdauer der durch die lichtdurchlässige Beschichtung geschützten Bauelemente tritt eine Senkung der Kosten ein.

Eine aus Teflon bestehende Beschichtung besitzt gute Gleiteigenschaften. Diese Beschichtung eignet sich besonders für Einsatzfälle, bei denen die Oberfläche der Einwirkung von klebrigen Partikeln wie Honigtau ausgesetzt ist, da diese Partikel von einer Teflon-Beschichtung besonders leicht entfernt werden können und der durch die Reinigung der Oberfläche hervorgerufene Verschleiß wesentlich reduziert werden kann.

Wird die Beschichtung aus mehr als einem Werkstoff gebildet und besteht die Beschichtung aus mehr als einer Schicht, lassen sich spezifische Eigenschaften der Beschichtungswerkstoffe wie zum Beispiel hohe Verschleißfestigkeit und Verhinderung von statischer Aufladung in gewünschter Weise kombinieren. Durch eine Beschichtung, die aus mehr als einer Werkstoffschicht besteht, sowie durch das Erzeugen der Beschichtung durch Bedampfung werden die Elastizitätseigenschaften und die Haftfähigkeit der Beschichtung verbessert. Es sind sehr dünne und damit lichtdurchlässige Schichtdicken erzielbar.

Mit einer gezielt nur auf die bei der Garnüberwachung optisch wirksamen Bereiche der Bauelemente aufgebrachten Beschichtung werden an bestimmten Stellen unerwünschte Oberflächeneigenschaften, wie zum Beispiel "elektrisch leitfähig" oder "isoliert" vermieden und die Menge des zur Beschichtung benötigten Materials gesenkt.

Die erfindungsgemäße Beschichtung erlaubt es, die Kosten bei der Herstellung von optisch wirksamen, lichtdurchlässigen Bauelementen zu senken. Zum Beispiel können Linsen aus relativ weichem Kunststoff mittels eines Preßvorganges gefertigt werden statt diese aus Rohlingen aus optischem Glas durch aufwendige Schleifvorgänge zu formen. Der Neigung des Kunststoffes zu statischer Aufladung und der damit verbundenen Anziehung von in der Luft schwebenden Staubpartikeln sowie der mangelhaften Härte des Kunststoffes, die eine unzureichende Widerstandsfähigkeit der Kunststoffoberfläche bei mechanischer Einwirkung, insbesondere beim Reinigen der Oberfläche, mit sich bringt, wird durch die Beschichtung wirksam begegnet.

Die erfindungsgemäße Beschichtung ist kostengünstig und kostensparend, im Hinblick auf die Verbesserung verschiedener Oberflächeneigenschaften universell nutzbar und vielseitig, beispielsweise an Lichtquellen, Lichtempfängern sowie Abdeckungen, einsetzbar. Insbesondere benötigt die Beschichtung praktisch keinen Platz beziehungsweise Raum, was bei einem Einsatz in Garnüberwachungseinrichtungen einer Vielstellen-Textilmaschine, zum Beispiel einer Rotorspinnmaschine, mit nur begrenzt zur Verfügung stehenden Bauraum von großem Vorteil ist.

Weitere Einzelheiten der Erfindung sind den Prinzipdarstellungen der Figuren entnehmbar.

Es zeigt:
- Fig. 1: eine optische Garnüberwachungsvorrichtung mit Bauelementen, die teilweise beschichtet sind,
- Fig. 2: eine weitere optische Garnüberwachungsvorrichtung, ebenfalls mit teilweise beschichteten Bauelementen.

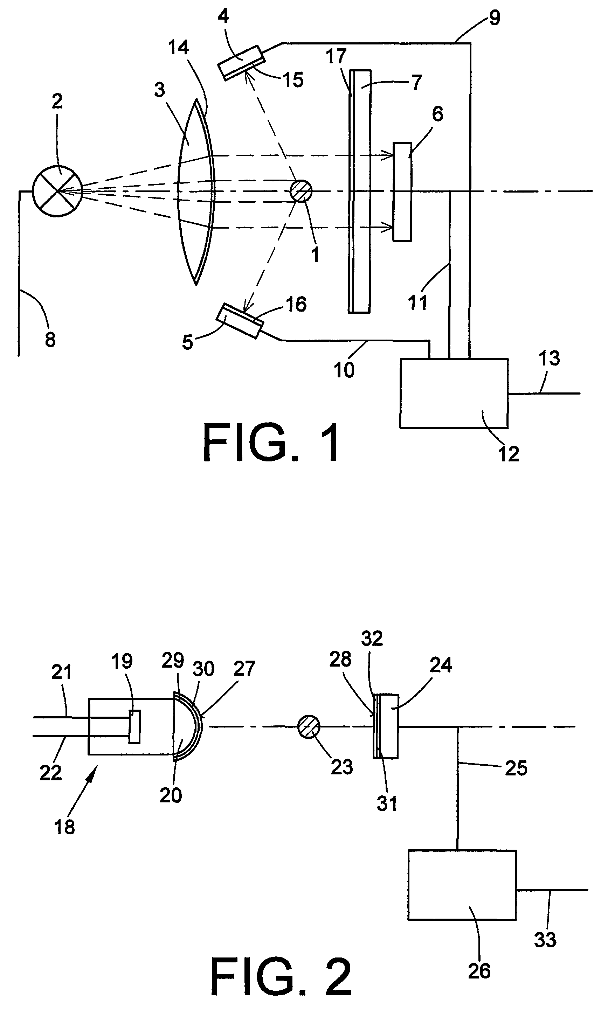

Die in Figur 1 dargestellte optische Garnüberwachungsvorrichtung, wie sie zum Beispiel an einer Spinnstelle einer Rotorspinnmaschine eingesetzt werden kann, ist zur Durchführung von Reflexlichtdetektion und Detektion der Abschattung ausgerüstet. Das Funktionsprinzip derartiger Meßeinrichtungen ist bekannt und wird daher hier nicht ausführlicher erläutert.

Zur Detektion von Eigenschaften des Garnes 1 weist die Vorrichtung zur optischen Garnüberwachung eine Lichtquelle 2, eine zwischen der Lichtquelle 2 und dem Garn 1 angeordnete Linse 3 und Sensoren 4, 5 für reflektiertes Licht sowie einen Sensor 6 für Restlicht auf. Das Garn 1 bewegt sich in Längsrichtung durch den Meßbereich und wird durch eine nicht dargestellte Führung im Meßbereich gehalten. Eine zwischen dem Garn 1 und dem Sensor 6 angeordnete Abdeckplatte 7 dient dem Schutz vor Verschmutzung des Sensors 6. Die Lichtquelle 2 ist über die Leitung 8 mit einer nicht dargestellten Spannungsquelle verbunden. Sowohl die Sensoren 4, 5 wie auch der Sensor 6 sind über Leitungen 9, 10, 11 mit einer Signalverarbeitungsanlage 12 verbunden. Die Leitung 13 dient zur Kommunikation der Signalverarbeitungsanlage 12 mit nicht dargestellten Datenverarbeitungsanlagen oder zur Steuerung eines ebenfalls nicht dargestellten Garnreinigers.

Linse 3, Sensor 4, 5 sowie Abdeckplatte 7 weisen an der dem Garn zugewandten Seite eine Beschichtung 14, 15, 16, 17 auf. Die Schichtdicke dieser Beschichtungen ist so gering, daß eine ausreichende Lichtdurchlässigkeit dieser Oberflächen gewährleistet ist. Diese geringe Schichtdicke ist mittels eines an sich bekannten Bedampfungsverfahrens erzeugt worden. Sowohl reflektiertes Licht als auch Restlicht, also Licht, das vom Garn oder auf andere Weise nicht abgedeckt oder reflektiert wird, trifft jeweils auf die Sensoren 4, 5, 6 auf und wird von diesen in elektrische Signale umgewandelt und in bekannter Weise weiterverarbeitet, zum Beispiel zur Ermittlung des Garndurchmessers oder zur Ermittlung von Farbabweichungen.

Schmutzpartikel werden im durch die Garnbewegung erzeugten Luftstrom mitgeführt und geraten dabei auch an die Oberflächen der Bauelemente der Garnüberwachungsvorrichtung. Durch die schmutzabweisenden Eigenschaften der Beschichtung 14, 15, 16, 17 wird einer Ablagerung der Schmutzpartikel auf den Oberflächen entgegengewirkt. Dadurch lassen sich die Reinigungsintervalle verlängern beziehungsweise die Zahl der Reinigungsvorgänge pro Zeiteinheit herabsetzen. Diese Reinigungsvorgänge im Rahmen vorbeugender, zustandsabhängiger oder störungsabhängiger Wartung dienen der Beseitigung der Schmutzpartikel, die sich auf die bei der Garnüberwachung eingesetzten, optisch wirksamen Oberflächen abgelagert haben.

Bei jedem der Reinigungsvorgänge besteht die Möglichkeit beziehungsweise die Gefahr, daß insbesondere durch härtere Schmutzpartikel die gereinigte Oberfläche verkratzt wird. Dieses kann durch die hohe Verschleißfestigkeit der Beschichtung 14, 15, 16, 17 erheblich gemindert oder sogar ganz verhindert werden. Das Verkratzen der Oberfläche üblicher Bauelemente kann mit zunehmender Nutzungsdauer ein solches Ausmaß erreichen, daß die erforderliche Lichtdurchlässigkeit der Oberfläche nicht mehr gewährleistet ist und das Bauelement ausgetauscht werden muß, um fehlerbehaftete Meßwerte zu vermeiden. Ein derartiger verschleißbedingter Austausch kann durch die erfindungsgemäße Beschichtung 14, 15, 16, 17 zumindest erheblich hinausgezögert werden. Sowohl durch die verlängerten Reinigungsintervalle als auch durch die verlängerte Nutzungsdauer der Bauelemente kann der Nutzungsgrad der Textilmaschine verbessert werden. Aus der verlängerten Nutzungsdauer ergeben sich zudem Kostenvorteile.

Eine besonders vorteilhafte Ausbildung der Erfindung zeigt die Figur 2. Zur Lichterzeugung in der Garnüberwachungsvorrichtung dient ein LED-Chip 18, der eine Leuchtdiode 19 und eine Linse 20 aufweist. Der LED-Chip 18 ist über Stromleiter 21, 22 an eine nicht dargestellte Spannungsquelle angeschlossen. Der als CCD-Zeilensensor ausgebildete Sensor 24 ist über die Leitung 25 mit einer Signalverarbeitungsanlage 26 verbunden. Die Kopplung der Signalverarbeitungsanlage 26 mit weiteren nicht dargestellten Datenverarbeitungsanlagen sowie gegebenenfalls das Ausführen von Steuerungsvorgängen erfolgt über die Leitung 33.

An der dem Garn 23 zugewandten Seite weisen der LED-Chip 18 und der Sensor 24 eine Beschichtung 27, 28 auf. Die Beschichtung 27, 28 besteht aus jeweils zwei Schichten 29, 30, 31, 32. Die untere Schicht 29, 31 besteht aus einem Keramikwerkstoff. Die obere Schicht 30, 32 besteht aus Teflon. Auch hier sind die Schichtdicken sehr gering.

Bei mechanischer Einwirkung auf die Oberfläche der Linse 20 oder des Sensors 24, zum Beispiel bei Reinigungsvorgängen, schützt spätestens die untere aus Keramikwerkstoff bestehende Schicht 29, 31 die darunterliegende Oberfläche. Dies ist besonders dann von Vorteil, wenn die Linse 20 aus einem relativ weichen Kunststoff besteht. Die Verwendung von Kunststoff erlaubt eine kostengünstige Herstellung der Linse 20 sowohl im Hinblick auf die Materialkosten wie auf die Fertigungskosten. Dabei ist die Linse 20 als Bauelement in dem LED-Chip 18 integriert und fest mit diesem zu einer Einheit verbunden.

Weitere Einsatzmöglichkeiten für die erfindungsgemäße Beschichtung bieten zum Beispiel die Oberflächen von Spiegeln, Prismen oder ähnlichen Bauelementen, die im Strahlengang einer Garnüberwachungsvorrichtung angeordnet sind, Lichtstrahlen in eine oder verschiedene Richtungen umlenken und einer ständigen Verschmutzung beziehungsweise Verflugung durch Staub, Faserfragmente, Avivage- und Schmutzpartikel ausgesetzt sind.

## Patentansprüche

1. Vorrichtung zur optischen Garnüberwachung mit mindestens einer Einrichtung, die der Minderung der Lichtdurchlässigkeit durch Umgebungseinflüsse auf Bauelemente wie Lichtquelle, Sensor oder Linse, entgegenwirkt, wobei die Einrichtung gegen unerwünschte Folgen von Umgebungseinflüssen eine lichtdurchlässige Beschichtung (14, 15, 16, 17, 27, 28) ist, die das Anlagern von die Lichtdurchlässigkeit mindernden Ablagerungen herabsetzt und den Verschleiß an der Oberfläche der Bauelemente infolge mechanischer Einwirkung, insbesondere beim Reinigen der Oberfläche, mindert,
und wobei mindestens die die Oberfläche bildende Schicht (14,15,16,17,30,32) der Beschichtung (14,15,16,17,27,28) aus Teflon besteht.

2. Vorrichtung nach Anspruch 1, **dadurch gekennzeichnet, daß** die Beschichtung (14, 15, 16, 17) aus mehr als einem Werkstoff gebildet ist.

3. Vorrichtung nach Anspruch 1 oder 2, **dadurch gekennzeichnet, daß** die Beschichtung (14, 15, 16, 17, 27, 28) aus mehr als einer Schicht besteht.

4. Vorrichtung nach einem der vorhergehenden Ansprüche, **dadurch gekennzeichnet, daß** die Beschichtung (14, 15, 16, 17, 27, 28) durch Bedampfung erzeugt ist.

5. Vorrichtung nach einem der vorhergehenden Ansprüche, **dadurch gekennzeichnet, daß** sich die Beschichtung (14, 15, 16, 17, 27, 28) nur über einen Teilbereich des Bauelementes erstreckt.

## Claims

1. An apparatus for visual yarn monitoring having at least one device which acts against the reduction in light transmission by environmental influences on components such as a light source, sensor or lens,
wherein the device for protection against undesired consequences of environmental influences is a transparent coating (14, 15, 16, 17, 27, 28) which reduces the settling of deposits which reduce light transmission and reduces the wear on the surface of the components as a result of mechanical action, in particular when cleaning the surface,
and wherein at least the upper layer (30, 32) of the coating (14, 15, 16, 17, 27, 28) is made from Teflon.

2. An apparatus according to Claim 1,
**characterised in that** the coating (14, 15, 16, 17) is made from more than one material.

3. An apparatus according to Claim 1 or 2,
**characterised in that** the coating (14, 15, 16, 17, 27, 28) consists of more than one layer.

4. An apparatus according to one of the preceding Claims, **characterised in that** the coating (14, 15, 16, 17, 27, 28) is produced by vapour deposition.

5. An apparatus according to one of the preceding Claims, **characterised in that** the coating (14, 15, 16, 17, 27, 28) extends only over a sub-region of the component.

## Revendications

1. Dispositif pour le contrôle optique d'un fil, comportant au moins un système contrecarrant la réduction de la translucidité, résultant d'influences de l'environnement sur des éléments structurels tels qu'une source de lumière, un capteur ou une lentille, sachant que le système, s'opposant à des conséquences indésirables d'influences de l'environnement, est un revêtement translucide (14, 15, 16, 17, 27, 28) qui diminue l'accumulation de dépôts réduisant la translucidité, et réduit l'usure se produisant à la surface des éléments structurels suite à une action mécanique, en particulier lors du nettoyage de ladite surface,
et sachant qu'au moins la couche supérieure (30, 32) du revêtement (14, 15, 16, 17, 27, 28) consiste en du Téflon.

2. Dispositif selon la revendication 1, **caractérisé par le fait que** le revêtement (14, 15, 16, 17) est constitué de plus d'un matériau.

3. Dispositif selon la revendication 1 ou 2, **caractérisé par le fait que** le revêtement (14, 15, 16, 17, 27, 28) comprend plus d'une couche.

4. Dispositif selon l'une des revendications précédentes, **caractérisé par le fait que** le revêtement (14, 15, 16, 17, 27, 28) est produit par vaporisation.

5. Dispositif selon l'une des revendications précédentes, **caractérisé par le fait que** le revêtement (14, 15, 16, 17, 27, 28) s'étend uniquement sur une région partielle de l'élément structurel.
